(19) Europäisches Patentamt
European Patent Office
Office européen des brevets

(11) Veröffentlichungsnummer: **0 009 547**
**A1**

# (12) EUROPÄISCHE PATENTANMELDUNG

(21) Anmeldenummer: **79102372.4**

(22) Anmeldetag: **11.07.79**

(51) Int. Cl.[3]: **C 07 C 103/38**
A 61 K 31/22
//C07C125/063

(30) Priorität: **14.08.78 AT 5913/78**

(43) Veröffentlichungstag der Anmeldung:
**16.04.80 Patentblatt 80/8**

(84) Benannte Vertragsstaaten:
**AT BE CH DE FR GB IT LU NL SE**

(71) Anmelder: **F.Hoffmann-La Roche & Co.**
***Aktiengesellschaft***
**Abt. VIII-Pt**
**CH-4002 Basel(CH)**

(72) Erfinder: **Reiner, Roland, Dr.**
**Rheinfelderstrasse 8**
**CH-4058 Basel(CH)**

(74) Vertreter: **Mezger, Wolfgang, Dr. et al,**
**Grenzacherstrasse 124 Postfach 601**
**CH-4002 Basel(CH)**

## (54) Fluoramphenicolester und diese enthaltende chemotherapeutische Präparate sowie deren Herstellung.

(57) Fluoramphenicolester der allgemeinen Formel

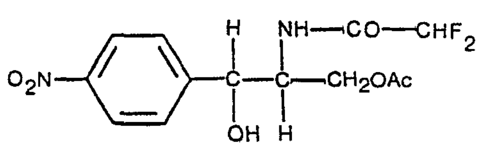

I

worin Ac den in vivo abspaltbaren Acylrest einer pharmakologisch verträglichen Carbonsäure bedeutet, und pharmakologisch verträgliche Salze solcher Ester, sowie deren Herstellung durch Veresterung von Fluoramphenicol und diese Verbindungen enthaltende chemotherapeutische Präparate.

EP 0 009 547 A1

Croydon Printing Company Ltd.

F. Hoffmann-La Roche & Co. Aktiengesellschaft, Basel, Schweiz

RAN 4410/129

Fluoramphenicolester und diese enthaltende chemotherapeutische Präparate sowie deren Herstellung.

Die Erfindung betrifft neue Fluoramphenicolester der allgemeinen Formel

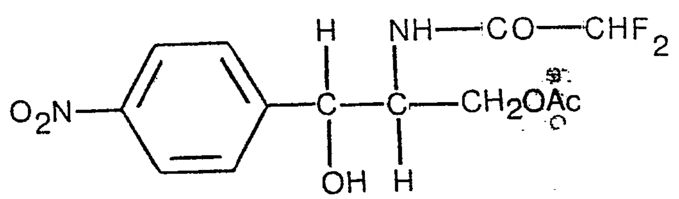

I

worin Ac den in vivo abspaltbaren Acylrest einer pharmakologisch verträglichen Carbonsäure bedeutet, und pharmakologisch verträgliche Salze solcher Ester.

Als Fluoramphenicol wird im Rahmen der vorliegenden Anmeldung die dem Chloramphenicol entsprechende Fluorverbindung, also das D-threo-1-(p-Nitrophenyl)-2-difluor-Mez/3.5.1979

acetamino-1,3-propandiol bezeichnet.

Als Acylreste Ac kommen generell die von den entsprechenden Chloramphenicolestern her bekannten Acylgruppen in Betracht.

Ac kann also beispielsweise der Acylrest

a) einer aliphatischen Monoaminomonocarbonsäure oder Monoaminodicarbonsäure (letztere in freier oder amidierter Form), je mit bis zu 6 C-Atomen, insbesondere der Acylrest der in Proteinen vorkommenden Säuren Alanin, Valin, Leucin, Isoleucin, Serin, Threonin, Asparaginsäure, Asparagin, Glutaminsäure, Glutamin, vorzugsweise jedoch von Glycin,

b) einer aliphatischen, keine Aminogruppen enthaltenden Dicarbonsäure mit bis zu 6 C-Atomen, wie Bernsteinsäure,

c) einer aromatischen Dicarbonsäure, insbesondere einer Benzoldicarbonsäure, wie Phthalsäure,

d) einer aliphatischen $C_{12-20}$-Monocarbonsäure, vorzugsweise Palmitinsäure oder Stearinsäure,

e) der Glykolsäure, deren Hydroxygruppe mit einer aliphatischen $C_{12-20}$-Monocarbonsäure, wie beispielsweise mit Stearinsäure, verestert ist (in welchem Falle Ac den Rest $-COCH_2OCOC_{17}H_{35}$ darstellt) oder

f) der Zimtsäure sein.

Die erfindungsgemässen Fluoramphenicolester der Formel I können dadurch erhalten werden, dass man Fluoramphenicol mit einem den Rest Ac abgebenden Veresterungsmittel behandelt, wobei Ac die obige Bedeutung besitzt, und das erhaltene Produkt gegebenenfalls in ein pharmakologisch verträgliches Salz überführt. Die Veresterung kann in an sich bekannter Weise

insbesondere in Analogie zur Herstellung der entsprechenden Chloramphenicolester erfolgen. So kann man beispielsweise zur Herstellung von Aminocarbonsäureestern, wie des Glycin- oder Alaninesters, als Veresterungsmittel die entsprechende, in der Aminogruppe geschützte Carbonsäure in Gegenwart eines Kondensationsmittels, beispielsweise eines Carbodiimids, und eines Lösungsmittels verwenden. Auf entsprechende Art lassen sich Veresterungen mit aliphatischen $C_{12-20}$-Monocarbonsäuren, wie Palmitinsäure, durchführen. Zur Herstellung der Halbester von aliphatischen oder aromatischen Dicarbonsäuren, wie des Monosuccinats oder des Monophthalats, kann man die entsprechenden Säureanhydride einsetzen. Auch Säurehalogenide können in üblicher Weise als Veresterungsmittel Verwendung finden, zweckmässig unter Zusatz organischer Basen, wie Pyridin.

Im Veresterungsmittel gegebenenfalls vorhandene Schutzgruppen, wie Aminoschutzgruppen, lassen sich nach durchgeführter Acylierung auf an sich bekannte Art wieder abspalten, beispielsweise mit verdünnten Säuren.

Erhaltene Ester mit basischen Gruppen, wie die in den Monoaminomonocarbonsäureestern vorhandenen Aminogruppen, oder mit sauren Gruppen, wie die in den Halbestern von Dicarbonsäuren vorhandenen Carboxylgruppen bilden mit Säuren bzw. mit Basen leicht wasserlösliche Salze (Säureadditionssalze bzw. Alkalimetallsalze und dergleichen).

Die erfindungsgemässen Fluoramphenicolester der Formel I zeichnen sich durch ein breites antibiotisches Wirkungsspektrum gegen grampositive und gramnegative Bakterien [inkl. gramnegative Anaerobier (Bacteroides)] sowie gegen Mycoplasmen, Rickettsien und grosse Viren aus. Wie die Ester des Chloramphenicols sind sie im Gegensatz zum unveresterten, bitter schmeckenden Diol praktisch geschmacklos und werden im Organismus hydrolytisch bzw. enzymatisch rasch unter Freisetzung des eigentlichen Wirkstoffs (Fluoramphenicol) gespalten. Gegenüber

dem bekannten Antibioticum Chloramphenicol bzw. den therapeutisch verwendeten Estern desselben, weisen die erfindungsgemässen Fluoramphenicolester in toxikologischer, chemotherapeutischer und/oder pharmakokinetischer Hinsicht Vorteile auf.

Für den Fluoramphenicol-3-glycinester (in Form des Hydrochlorids) können beispielsweise die folgenden Werte angegeben werden:

Akute Toxizität (Maus): $LD_{50}$ = 2000-4000 mg/kg s.c., > 5000 mg/kg p.o.

In-vivo-Aktivität (Maus) ($CD_{50}$ p.o.): 27 mg/kg gegen Streptococcos pyogenes, 23 mg/kg gegen Escherichia coli, 47 mg/kg gegen Pseudomonas aeruginosa BA, 12 mg/kg gegen Salmonella typhimurium.

Der Ester wird bei physiologischem pH sofort hydrolytisch zu wirksamem Fluoramphenicol gespalten, welches im Gegensatz zu Chloramphenicol metabolisch beständig ist.

Die erfindungsgemässen Fluoramphenicolester können auf Grund ihrer antibiotischen Wirkung als Chemotherapeutika besonders zur Behandlung von Typhus, Paratyphus, Meningitis, Peritonitis, schweren Luftwegs- sowie Harnwegsinfektionen (peroral, parenteral, rektal) oder als Desinfektionsmittel Verwendung finden, wobei die Art der galenischen Formulierung (Tabletten, Kapseln, Suspensionen, Injektionslösungen, Suppositorien) und der medizinischen Applikation der Formulierung bzw. der Applikation der bekannten Chloramphenicolester entsprechen kann. Als Dosierung kommen für Erwachsene bei oraler oder parenteraler Verabreichung Mengen von ca. 2 bis 3 g pro Tag in 3 Einzelgaben in Betracht.

## Beispiel 1

Herstellung von Fluoramphenicol-3-(glycinester)-hydrochlorid

11,6 g Fluoramphenicol, 7,0 g N-t-Butyloxycarbonyl-glycin und 3,2 g Pyridin werden in 100 ml Acetonitril gelöst. Diese Lösung wird bei 20°C mit einer Lösung von 8,2 g Dicyclohexylcarbodiimid in 100 ml Acetonitril versetzt. Das Reaktionsgemisch wird 16 Stunden bei 20°C stehen gelassen. Nach Abfiltrieren des Dicyclohexyl-harnstoffes wird das Filtrat unter vermindertem Druck eingedampft. Der resultierende Rückstand wird in Aethylacetat gelöst und unter Eiszusatz nacheinander mit 1N Schwefelsäure und mit 10%iger Natriumhydrogencarbonat-Lösung gewaschen. Die Aethylacetatlösung wird dann über Magnesiumsulfat getrocknet und unter vermindertem Druck eingedampft. Der verbleibende harzige Rückstand (17,0 g) wird an einer Kieselgel-Säule mit Benzol/Essigester (1:5) als Elutionsmittel chromatographiert, wobei 6,2 g Fluoramphenicol-3-(N-t-butyloxycarbonyl)-glycinester in reiner Form als farbloser Schaum erhalten werden.

6,2 g Fluoramphenicol-3-(N-t-butyloxycarbonyl)-glycinester werden in 200 ml einer 1,5N Lösung von Salzsäure in Eisessig gelöst und 30 Minuten stehen gelassen. Das Reaktionsgemisch wird unter vermindertem Druck eingeengt und mit Diäthyläther versetzt, wobei die gewünschte Substanz ausfällt. Diese wird dann aus Aethanol-Diäthyläther umgefällt, abgenutscht, mit Aether gewaschen und bei 20°C unter vermindertem Druck getrocknet. Man erhält 4,4 g Fluoramphenicol-3-(glycinester)-hydrochlorid als farbloses Pulver. Schmelzpunkt oberhalb 120°C (Zersetzung). $[\alpha]_D^{20} = +21,4^\circ$ (c = 0,986 in Methanol).

Beispiel 2

Herstellung von Fluoramphenicol-3-(L-alaninester)-hydrochlorid

11,6 g Fluoramphenicol und 7,56 g N-t-Butyloxycarbonyl-L-alanin werden in 100 ml Tetrahydrofuran gelöst. Zu dieser Lösung wird eine Lösung von 8,2 g Dicyclohexyl-carbodiimid in 100 ml Tetrahydrofuran hinzugefügt und das resultierende Reaktionsgemisch über Nacht bei 25°C stehen gelassen. Darauf wird vom ausgefallenen Dicyclohexyl-harnstoff (5,3 g) abfiltriert und das Filtrat unter vermindertem Druck bei 40°C eingedampft. Der verbleibende kristalline Rückstand wird in 1000 ml Essigester gelöst. Diese Lösung wird zweimal mit 10%iger Natriumcarbonatlösung sowie einmal mit Wasser gewaschen, über Natriumsulfat getrocknet und anschliessend unter vermindertem Druck bei 40°C eingedampft. Der Eindampfrückstand wird aus 150 ml Aethanol umkristallisiert, wobei 7,2 g reiner Fluoramphenicol-3-(N-t-butyloxycarbonyl)-L-alaninester vom Schmelzpunkt 211-213°C (Zersetzung) erhalten werden.

5 g Fluoramphenicol-3-(N-t-butyloxycarbonyl)-L-alaninester werden in 200 ml einer 2N Lösung von Chlorwasserstoff in Dioxan gelöst und 30 Minuten bei 25°C gerührt. Die Reaktionslösung wird danach bei 35°C unter vermindertem Druck eingedampft und der resultierende harzige Rückstand aus Aethanol-Diäthyläther umgefällt. Man erhält somit 3,9 g sehr gut wasserlösliches Fluoramphenicol-3-(L-alaninester)-hydrochlorid als farbloses amorphes Pulver. Schmelzpunkt oberhalb 120°C (Zersetzung). $[\alpha]_D^{20} = +3,1^{\circ}$ (c = 1,000 in Methanol).

Beispiel 3

Herstellung von Fluoramphenicol-3-palmitat

11,6 g Fluoramphenicol und 10,25 g Palmitinsäure werden in 100 ml Tetrahydrofuran gelöst. Zu dieser Lösung wird eine Lösung von 8,2 g Dicyclohexyl-carbodiimid in 100 ml Tetrahydrofuran

hinzugefügt und das resultierende Reaktionsgemisch über Nacht bei 25°C stehen gelassen. Darauf wird vom ausgefallenen Dicyclohexyl-harnstoff abfiltriert und das Filtrat unter vermindertem Druck bei 40°C eingedampft. Der Eindampfrückstand wird in 1000 ml Essigester gelöst. Diese Lösung wird zweimal mit 10%iger Natriumcarbonatlösung und einmal mit Wasser gewaschen, über Natriumsulfat getrocknet und anschliessend unter vermindertem Druck bei 40°C eingedampft. Der Eindampfrückstand wird aus Aethanol umkristallisiert, wobei 4,5 g Fluoramphenicol-3-palmitat als in Wasser praktisch unlösliche, vollkommen geschmacklose und farblose Substanz erhalten werden. Schmelzpunkt 94-96°C. $[\alpha]_D^{20} = +10,2°$ (c = 1,000 in Methanol).

## Beispiel 4

### Herstellung von Fluoramphenicol-3-monosuccinat-natriumsalz

8,7 g Fluoramphenicol und 3,0 g Bernsteinsäureanhydrid werden in 100 ml Tetrahydrofuran 6 Stunden unter Rückfluss gekocht. Danach wird die Reaktionslösung unter vermindertem Druck eingedampft. Der verbleibende Rückstand wird in 200 ml Diäthyläther gelöst, von wenig Ungelöstem abfiltriert und das Filtrat mit 25 ml einer 2N Lösung von 2-Aethylcapronsäure-Natriumsalz in Essigester versetzt. Die dabei ausgefallene Substanz wird abfiltriert und mit Diäthyläther gewaschen. Danach wird diese Substanz in wenig Wasser gelöst und zweimal mit Essigester extrahiert. Die Essigesterextrakte werden verworfen. Die wässrige Phase wird im Hochvakuum eingedampft. Der resultierende Rückstand wird einmal mit Isopropanol abgedampft, darauf in Essigester gelöst, von etwas Ungelöstem abfiltriert und das Filtrat mit Diäthyläther verdünnt. Die dabei ausgefallene Substanz wird abgenutscht, mit Diäthyläther gewaschen und im Hochvakuum bei 50°C getrocknet. Man erhält so 2,2 g Fluoramphenicol-3-monosuccinat-natriumsalz als farbloses, sehr gut wasserlösliches Pulver. Schmelzpunkt oberhalb 100°C (Zersetzung). $[\alpha]_D^{20} = +4,42°$ (c = 0,770 in Wasser).

## Beispiel 5

Es wird in üblicher Weise eine Gelatine-Steckkapsel folgenden Inhaltes hergestellt:

| | |
|---|---|
| Fluoramphenicol-3-(glycinester)-hydrochlorid | 666 mg |
| Luviskol | 26 mg |
| Mannit | 26 mg |
| Talk | 20 mg |
| Magnesiumstearat | 3 mg |

## Beispiel 6

Es wird in üblicher Weise ein Lyophilisat folgender Zusammensetzung hergestellt:

| | |
|---|---|
| Fluoramphenicol-3-(glycinester)-hydrochlorid | 666 mg |
| p-Hydroxybenzoesäure-methylester | 0,6 mg |
| p-Hydroxybenzoesäure-propylester | 0,06 mg |

Dieses Lyophilisat wird in eine Ampulle abgefüllt. Eine derartige Ampulle enthält die lyophilisierten Wirkstoffe für 6 ml spritzfertiger Lösung. Zur Herstellung von 6 ml spritzfertiger Lösung werden dem Lyophilisat 5,5 ml destilliertes Wasser zugesetzt.

## Beispiel 7

Es wird in üblicher Weise ein Lyophilisat folgender Zusammensetzung hergestellt:

| | | |
|---|---|---|
| Fluoramphenicol-3-monosuccinat-natriumsalz | 710 | mg |
| p-Hydroxybenzoesäure-methylester | 0,65 | mg |
| p-Hydroxybenzoesäure-propylester | 0,065 | mg |

Dieses Lyophilisat wird in eine Ampulle abgefüllt. Eine derartige Ampulle enthält die lyophilisierten Wirkstoffe für 7 ml spritzfertiger Lösung. Zur Herstellung von 7 ml spritzfertiger Lösung werden dem Lyophilisat 6,4 ml destilliertes Wasser zugesetzt.

## Beispiel 8

Es wird in üblicher Weise eine Gelatine-Steckkapsel folgenden Inhaltes hergestellt:

| | |
|---|---|
| Fluoramphenicol-3-palmitat | 910 mg |
| Luviskol | 36 mg |
| Mannit | 36 mg |
| Talk | 27 mg |
| Magnesiumstearat | 4 mg |

Patentansprüche

1. Fluoramphenicolester der allgemeinen Formel

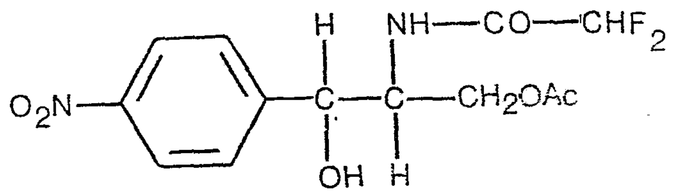

I

worin Ac den in vivo abspaltbaren Acylrest einer pharmakologisch verträglichen Carbonsäure bedeutet,

und pharmakologisch verträgliche Salze solcher Ester.

2. Fluoramphenicolester der Formel I gemäss Anspruch 1 und deren pharmakologisch verträgliche Salze, worin Ac der Acylrest

- einer aliphatischen Monoaminomonocarbonsäure oder Monoaminodicarbonsäure (letztere in freier oder amidierter Form), je mit bis zu 6 C-Atomen,

- einer aliphatischen, keine Aminogruppen enthaltenden Dicarbonsäure mit bis zu 6 C-Atomen,

- einer aromatischen Dicarbonsäure,

- einer aliphatischen $C_{12-20}$-Monocarbonsäure,

- der Glykolsäure, deren Hydroxygruppe mit einer aliphatischen $C_{12-20}$-Monocarbonsäure verestert ist oder

- der Zimtsäure ist.

3. Fluoramphenicol-3-glycinester und dessen pharmakologisch verträgliche Säureadditionssalze.

4. Fluoramphenicol-3-glycinester-hydrochlorid.

5. Fluoramphenicol-3-(L-alaninester) und dessen pharmakologisch verträgliche Säureadditionssalze.

6. Fluoramphenicol-3-(L-alaninester)-hydrochlorid.

7. Fluoramphenicol-3-palmitat.

8. Fluoramphenicol-3-monosuccinat und dessen pharmakologisch verträgliche Salze mit Basen.

9. Fluoramphenicolester gemäss einem der Ansprüche 1-8 als pharmazeutische Wirkstoffe.

10. Verfahren zur Herstellung von Fluoramphenicolestern der allgemeinen Formel

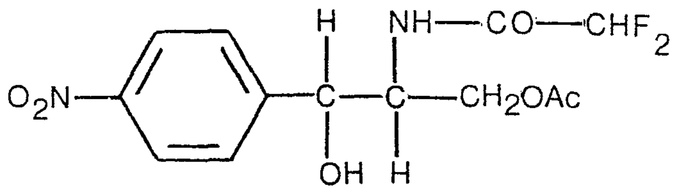

I

worin Ac den in vivo abspaltbaren Acylrest einer pharmakologisch verträglichen Carbonsäure bedeutet,
und von pharmakologisch verträglichen Salzen solcher Ester, dadurch gekennzeichnet, dass man Fluoramphenicol mit einem den Rest Ac abgebenden Veresterungsmittel behandelt und dass man gegebenenfalls das erhaltene Produkt in ein pharmakologisch verträgliches Salz überführt.

11. Verfahren nach Anspruch 10, dadurch gekennzeichnet, dass Ac der Acylrest

- einer aliphatischen Monoaminomonocarbonsäure oder Monoaminodicarbonsäure (letztere in freier oder amidierter Form), je mit bis zu 6 C-Atomen,

- einer aliphatischen, keine Aminogruppen enthaltenden Dicarbonsäure mit bis zu 6 C-Atomen,

- einer aromatischen Dicarbonsäure,

- einer aliphatischen $C_{12-20}$-Monocarbonsäure,

- der Glykolsäure, deren Hydroxygruppe mit einer aliphatischen $C_{12-20}$-Monocarbonsäure verestert ist oder

- der Zimtsäure ist.

12. Chemotherapeutische Präparate, enthaltend einen Fluoramphenicolester der Formel I gemäss Anspruch 1 oder ein pharmakologisch verträgliches Salz eines solchen Esters.

***

0009547

Nummer der Anmeldung

Europäisches Patentamt

# EUROPÄISCHER RECHERCHENBERICHT

EP 79 102 372.4

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | JOURNAL OF MEDICINAL CHEMISTRY, Band 16, Nr. 8, 1973 New York E.R. GARRETT et al. "Structure-Activity Relationship of Chloramphenicols" Seiten 917 bis 922 -- | | C 07 C 103/38<br>A 61 K 31/22<br>//C 07 C 125/063 |
| A | THE MERCK INDEX achte Auflage 1968, MERCK CO. Rahway, U.S.A. Seite 233 -- | | RECHERCHIERTE SACHGEBIETE (Int. Cl.3)<br>A 61 K 31/22<br>C 07 C 103/38 |
| A | US - A - 3 190 910 ( E. D. NICOLAIDES) -- | | |
| A | US - A - 2 538 764 ( H. M. CROOKS et al.) ---- | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 09-01-1980 | STOOS |